# EUROPEAN PATENT APPLICATION

(11) **EP 4 623 924 A1**
(43) Date of publication of application: **01.10.2025**
(21) Application number: 23905489.3
(22) Date of filing: 31.10.2023
(51) Int. Cl.: A61K 36/9064, A61K 36/8994, A61K 36/8945, A61K 36/62, A61K 36/484, A61K 36/48, A61K 36/346, A61K 36/284, A61K 36/258, A61K 36/076, A61P 31/14, A61P 11/00

(54) **USE OF SHENLING BAIZHU IN PREPARING MEDICAMENT FOR TREATING POST-HEALING SEQUELAE OF NOVEL CORONAVIRUS INFECTED PERSON**

(30) Priority: 21.12.2022 CN 202211652297
(71) Applicant: Beijing Handian Pharmaceutical Co., Ltd., Beijing 101500 (CN)
(72) Inventor: LIN, Deliang, Beijing 100020 (CN)
(74) Representative: Baldus, Oliver
(86) International application number: PCT/CN2023/128850
(87) International publication number: WO 2024/131318

(57) **Abstract**

Provided is use of Shenling Baizhu in preparing a medicament for treating post-healing sequelae of a novel coronavirus infected person. The sequela is selected from at least one of shortness of breath, fatigue and weakness, inappetence, and diarrhea. The Shenling Baizhu is prepared from the following raw materials in parts by weight: 400 parts of ginseng, 400 parts of poria cocos, 400 parts of Rhizoma Atractylodis macrocephalae stir-fried with bran, 400 parts of Chinese yam, 300 parts of fried white hyacinth beans, 200 parts of lotus seeds, 200 parts of coix seeds stir-fried with bran, 200 parts of Fructus amomi, 200 parts of Platycodon grandiflorum, and 400 parts of liquorice.

## Description

### CROSS-REFERENCE TO RELATED APPLICATION

The present disclosure claims the priority to the Chinese patent application No. 202211652297.0 filed on December 21, 2022 and entitled "USE OF SHENLING BAIZHU IN TREATING POST-HEALING SEQUELAE OF NOVEL CORONAVIRUS INFECTED PERSON, AND IMPROVING QUALITY OF LIFE", the contents of which are incorporated herein by reference in entirety.

### TECHNICAL FIELD

The present disclosure belongs to the field of medicine and traditional Chinese medicine, and in particular relates to the application of Shenling Baizhu in treating the post-healing sequelae of novel coronavirus infected person, especially in addressing sequelae in patients recovering from pneumonia caused by the novel coronavirus and in improving quality of life.

### BACKGROUND ART

Novel coronavirus pneumonia (i.e., Corona Virus Disease 2019, COVID-19, which is also referred to as "COVID-19" hereinafter) is one of the most severe public health security incidents to have broken out in this century. According to the latest real-time statistics from the World Health Organization, as of December 5, 2022, the cumulative number of confirmed COVID-19 cases worldwide has exceeded 644 million, with 6.652 million cumulative deaths. According to domestic and international literature reports, the novel coronavirus not only damages the respiratory system, but also affects the lung, heart, liver, kidney, hematological system, and reproductive system, etc. It causes serious damage to multiple systems of patients during the post-healing and recovery period, with a series of sequelae including weakness, cough, shortness of breath, anorexia, low-grade fever, night sweating, constipation or diarrhea, oppression in chest, palpitation, chronic hemorrhage, sensory disturbance, and dyskinesia. In January, 2021, the Lancet, the world's top medical journal, published a study on the follow-up of 1,733 discharged patients with COVID-19, which was conducted by multiple centers including Wuhan Jinyintan Hospital. The results showed that more than 70% (76%) of the patients had at least one symptom after 6 months of acute infection. Fatigue or muscle weakness (63%) and sleep disorder (26%) are the most common symptoms (Huang C, et al. 6-month consequences of COVID-19 in patients discharged from hospital: a Cohort study [J]. Lancet. 2021; 397(10270):220-232). The above-mentioned sequelae greatly affect the quality of life of post-healing patients with COVID-19 and delay the complete recovery of patients, making it difficult for the recovery of the immunity of the patient, thus laying the risk of re-infection. According to the prediction from the experts, the novel coronavirus has undergone continuous mutation, and although its virulence has decreased, its infectiousness has increased, making its transmission more secretive. The novel coronavirus will coexist with humans for a long time, and patients with pneumonia caused by the novel coronavirus will continue to appear in the population. Therefore, there is a continuing clinical demand for medicaments capable of treating sequelae in clinically cured COVID-19 patients and improving their quality of life.

The Shenling Baizhu Granules produced by Beijing Handian Pharmaceutical Co., Ltd. is the only Shenling Baizhu Granules (National Drug Approval No. Z20040416) approved by the former National Food and Drug Administration in China, with a specification of 3g per sachet and a daily dosage of crude drugs being 27.9g. This specification of Shenling Baizhu Granules is an exclusive product of Handian. Other Shenling Baizhu Granules available on the market are homonymous but different formulae or homonymous but different substances, with a specification of 6g per sachet and a daily dosage of crude drugs being 13.95g. The production process of Beijing Handian Pharmaceutical Co., Ltd. includes four parts: extraction of volatile oil, extraction by water, extraction by ethanol and incorporation of raw yam powder into the formulation. The production process of other granule manufacturers includes two parts: extraction of volatile oil and extraction by water. The Shenling Baizhu powder/pills both use raw powder as the medicinal material, which differs significantly in material basis from the Shenling Baizhu Granules produced by Beijing Handian Pharmaceutical Co., Ltd. There are also substantial differences in the formulation and preparation processes. Other Shenling Baizhu Granules implement 1998 Ministry-issued standard, and are made of 1 part clear paste, 2 parts sucrose, and 2.5 parts dextrin. The Shenling Baizhu Granules produced by Beijing Handian Pharmaceutical Co., Ltd. implement the 2005 version of the national standard and are made of 1part clear paste and 0.4 part lactose, which greatly reduces the excipient intake of patients and increases patient compliance.

The unique process of Shenling Baizhu Granules by Beijing Handian Pharmaceutical Co., Ltd., based on the substance foundation of drug efficacy, has been granted a national invention patent with the Patent No. ZL200510086669.8. To alleviate post-healing sequelae in pneumonia patients caused by the novel coronavirus, help patients achieve early and complete health restoration, and return to normal production and life, the inventors trialed the Shenling Baizhu Granules produced by Beijing Handian Pharmaceutical Co., Ltd. for treating the sequelae after recovery from pneumonia caused by novel coronavirus, thus achieving significant therapeutic effects.

### SUMMARY

In view of the shortcomings of the prior art, the present disclosure provides a new medical use of Shenling Baizhu, especially a Shenling Baizhu Granule (National Drug Approval No. Z20040416) produced by Beijing Handian Pharmaceutical Co., Ltd., in treating the post-healing sequelae of novel coronavirus pneumonia patients and improving the quality of life after recovery.

In order to achieve the above technical effect, the following technical solutions are adopted in the present disclosure.

An application of Shenling Baizhu in the preparation of a pharmaceutical for treating post-healing sequelae of novel coronavirus pneumonia patients and improving the quality of life is provided. The sequela is selected from at least one of shortness of breath, fatigue and weakness, inappetence, and diarrhea. The raw materials of Shenling Baizhu are composed of the following parts by weight of Chinese medicinal herbs:

400 parts of ginseng, 400 parts of poria cocos, 400 parts of rhizoma atractylodis macrocephalae stir-fried with bran, 400 parts of Chinese yam, 300 parts of fried white hyacinth beans, 200 parts of lotus seeds, 200 parts of coix seeds stir-fried with bran, 200 parts of fructus amomi, 200 parts of platycodon grandiflorum, and 400 parts of liquorice.

Optionally, the sequelae further include at least one selected from vomiting/nausea, abdominal distention and fullness, and weak bowel movements.

Optionally, the term "improving the post-healing life quality of novel coronavirus pneumonia patients" means that after the Shenling Baizhu is applied to novel coronavirus pneumonia patients, the score of SF12 scale of the patients is significantly decreased compared with the score before treatment, and the P value is at least less than 0.05.

Optionally, the medicament disclosed in the present disclosure is a clinically acceptable preparation.

Optionally, the medicament is selected from decoction, pill, powder, granule, tablet or capsule.

As an optional embodiment, the medicament is a granule, which is prepared by the following steps, including:
I. taking each Chinese medicinal herb according to the parts by weight;
II. pulverizing Chinese yam into fine powder, and sieving with a 100-mesh sieve for further use;
III. extracting volatile oil from fructus amomi by steam distillation until exhaustion, and collecting the distilled aqueous solution in another container; soaking the spent fructus amomi residue together with ginseng and rhizoma atractylodis macrocephalae stir-fried with bran in 4-5 times their weight of 95% ethanol for 24 hours, then heating under refluxing for 3 hours, filtering, recovering ethanol from the filtrate, and concentrating to a clear paste with a relative density of 1.00-1.15 at 65°C, so as to obtain an alcohol extract A; reflux-extracting the residue again with 4-5 times their weight of 50% ethanol for 3 hours, filtering, recovering ethanol from the filtrate, and concentrating to a clear paste with a relative density of 1.12-1.13 at 80°C, so as to obtain an alcohol extract B; and reserving the ethanol-extracted residue for further use;
IV. decocting poria cocos, fried white hyacinth beans, lotus seeds, coix seeds stir-fried with bran, platycodon grandiflorum, and liquorice with 8-10 times their weight of water for 2 hours, then filtering; combining the residue with the ethanol-extracted residue reserved in Step III, then decocting again with 8 times their weight of water for 1.5 hours, and filtering; combining the two filtrates and the distilled aqueous solution obtained in Step III, standing, and filtering; concentrating the filtrate to a relative density of 1.20 at 80°C, then adding the alcohol extract A and alcohol extract B, and continuing to concentrate into a clear paste with a relative density of 1.30-1.34 at 80°C;
V. blending the fine powder of Chinese yam obtained in step II into the clear paste obtained in step IV, drying at a low temperature of 60°C to obtain a dry paste, and then pulverizing, so as to obtain medicinal powder; and
VI. taking 1 part by weight of the medicinal powder and 0.4 part by weight of the lactose powder obtained in step V, mixing them evenly, granulating with 70%-75% ethanol, drying and then sizing; and spraying the volatile oil of fructus amomi obtained in step III, mixing them evenly, and processing into 1000g, so as to obtain the product.

As an optional embodiment, when the above granules are applied to a subject in need, the dosage is 3 times a day, 3g per dose, which is converted into a crude drug dosage of 27.9g/day.

Through clinical observation, Shenling Baizhu Granule can effectively treat the post-healing sequelae of novel coronavirus pneumonia patients, so as to improve the quality of life.

### BRIEF DESCRIPTION OF THE DRAWINGS

The present disclosure is further described in conjunction with the drawings:

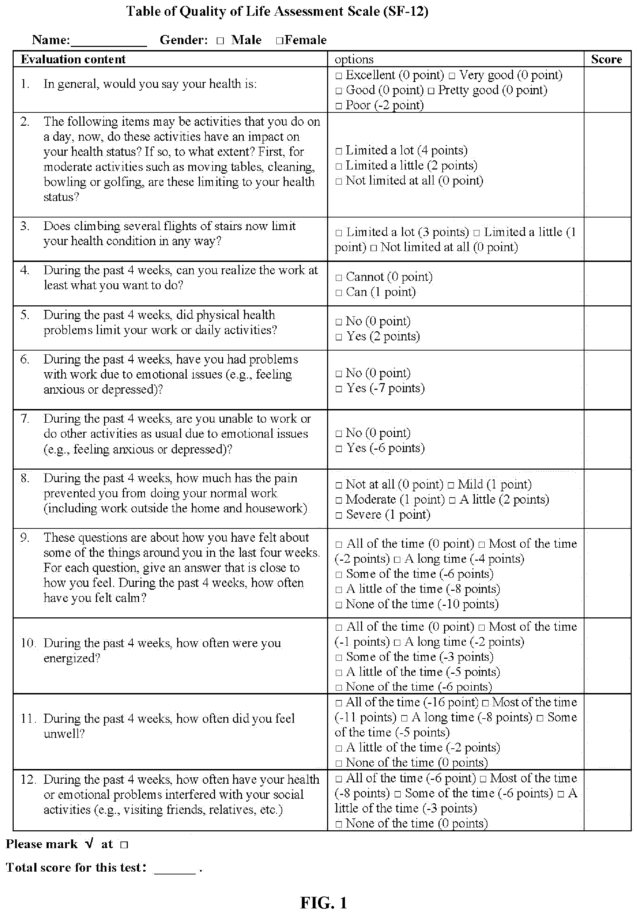

FIG.1 illustrates a sample table of the quality of life assessment scale (SF-12).

### DETAILED DESCRIPTION OF EMBODIMENTS

2019 novel coronavirus (2019-nCoV) not only causes serious damage to the respiratory system of patients in the infectious period, but also has a great impact on the whole biological system of patients, which brings lasting and long-term sequelae to patients and greatly affects their health and quality of life. The inventors conducted clinical observation tests on the influence of Shenling Baizhu preparation on the post-healing life quality of novel coronavirus pneumonia patients in four hospitals: Tongshan People's Hospital, Dongfeng General Hospital of Sinopharm, Xianning Central Hospital and Yangxin People's Hospital. After nearly one month of intervention treatment, the symptoms such as shortness of breath, fatigue and weakness, inappetence and diarrhea have been significantly improved, and the quality of life has been significantly improved.

The present disclosure will be described below with reference to specific examples. Those skilled in the art can understand that these examples are only used to illustrate the present disclosure, and they do not limit the scope of the present disclosure in any way.

The experimental methods in the following examples are all conventional methods, unless otherwise specified. Unless otherwise specified, the medicinal raw materials and reagent materials used in the following examples are all commercially available products.

In the field of traditional Chinese medicine, there are special terms to describe clinical symptoms. For example, "physical fatigue and lack of strength" is termed as "fatigue and weakness", "inappetence" is termed as "poor appetite", "diarrhea" is termed as "loose and soft stools", and so on. In the following examples, relevant clinical symptoms will uniformly employ standard traditional Chinese medicine terminology.

### Example l: Preparation of Shenling Baizhu Granules

Prescription: 400 parts of ginseng, 400 parts of poria cocos, 400 parts of rhizoma atractylodis macrocephalae stir-fried with bran, 400 parts of Chinese yam, 300 parts of fried white hyacinth beans, 200 parts of lotus seeds, 200 parts of coix seeds stir-fried with bran, 200 parts of fructus amomi, 200 parts of platycodon grandiflorum, and 400 parts of liquorice.

### Production process:

I. Each Chinese medicinal herb was taken according to the parts by weight.
II. Chinese yam was pulverized into fine powder and sieved with a 100-mesh sieve for further use.
III. The volatile oil was extracted from fructus amomi by steam distillation until exhaustion, and the distilled aqueous solution was collected in another container. The spent fructus amomi residue was soaked together with ginseng and rhizoma atractylodis macrocephalae stir-fried with bran in 4-5 times their weight of 95% ethanol for 24 hours, then the resultant was heated under refluxing for 3 hours and then filtered, the ethanol was recovered from the filtrate, and the resultant was concentrated to a clear paste with a relative density of 1.00-1.15 at 65°C, so as to obtain an alcohol extract A. The residue was reflux-extracted again with 4-5 times their weight of 50% ethanol for 3 hours and then filtered, the ethanol was recovered from the filtrate, and the resultant was concentrated to a clear paste with a relative density of 1.12-1.13 at 80°C, so as to obtain an alcohol extract B. The ethanol-extracted residue was reserved for further use.
IV. Poria cocos, fried white hyacinth beans, lotus seeds, coix seeds stir-fried with bran, platycodon grandiflorum, and liquorice were decocted with 8-10 times their weight of water for 2 hours and then filtered. The residue was combined with the ethanol-extracted residue reserved in Step III, then the resultant was decocted again with 8 times their weight of water for 1.5 hours and then filtered. The two filtrates were combined with the distilled aqueous solution obtained in Step III, the resultant was stood and filtered. The filtrate was concentrated to a relative density of 1.20 at 80°C, then the alcohol extract A and alcohol extract B were added, and the mixture was further concentrated to a clear paste with a relative density of 1.30-1.34 at 80°C.
V. The fine powder of Chinese yam obtained in step II was blended into the clear paste obtained in step IV, the resultant was dried at a low temperature of 60°C to obtain a dry paste, and then the dry paste was pulverized, so as to obtain medicinal powder.
VI. 1 part by weight of the medicinal powder and 0.4 part by weight of the lactose powder obtained in step V were taken and mixed evenly, the resultant was granulated with 70%-75% ethanol and then dried and sized. The volatile oil of fructus amomi obtained in step III was sprayed, and then the resultant was mixed evenly and processed into 1000g and packaged in portions of 3 g per sachet.

Dosage and administration: three times a day, each time 1 sachet.

### Example 2: Intervention effect of Shenling Baizhu Granule on the post-healing life quality of novel coronavirus pneumonia patients

The present example was carried out in Tongshan People's Hospital, Dongfeng General Hospital of Sinopharm, Xianning Central Hospital and Yangxin County People's Hospital in Hubei Province from September 2021 to July 2022.

### 1. Distribution of cases

A total of 313 subjects were enrolled in the study, and 313 cases were completed, accounting for 100% of the enrolled cases. Eight cases over the age of 18-80 were excluded and 305 cases entered the analysis set.

### 2. General information of cases

Male 166 cases (54.43%), female 139 cases (45.57%); Han ethnicity 303 cases (99.34%); mean age 46.34±14.09 years, with the youngest being 2 years old and the oldest 84 years old. Mean body weight 63.41±7.51 kg.

### 3. Vital signs

At the beginning of the study, vital signs, including body temperature, respiration, blood pressure, and heart rate, were all within normal ranges. No clinically significant abnormalities in these parameters were observed during the study period.

### 4. Dosage Regimen

The mean medication duration during the study period was 28.09±0.88 days, with the shortest medication duration of 25 days, the longest medication duration of 32 days, and the median medication duration of 28 days. The day before the subjects started taking medication was recorded as Day 0, the first day of taking medication as Day 1, and so forth until Day 28.

The specific dosing regimen was: Shenling Baizhu Granules, 3 times a day, 1 sachet (3g/sachet) per dose, for 28 consecutive days. The Shenling Baizhu Granules were produced by Beijing Handian Pharmaceuticals Co., Ltd., and the production process is the same as that described in Example 1, with batch numbers of 211032, 211134, and 211232.

### 5. Efficacy evaluation indexes

The main efficacy index is the disappearance rate of shortness of breath, fatigue and weakness, anorexia, and loose and soft stools, and the secondary index is measured by the SF-12 (Table of quality of life assessment scale, see FIG. l)

### 6. Data acquisition

All the subjects visited the hospital on day 0, day 14 and day 28, respectively, in order to collect the symptoms and scoring records, in which the data of day 0 was recorded as "visit 1", the data of day 14 as "visit 2" and the data of day 28 as "visit 3", and the three groups of data were statistically analyzed.

### 7. Statistics and analysis

Statistical analysis was performed using SAS 9.4 (or above version) statistical analysis software.

All statistical tests were conducted by two-sided test, and the test statistics and their corresponding P values were given, where P<0.05 can be considered as statistically significant.

For quantitative data, the number of cases, mean, standard deviation, minimum, median, maximum, upper quartile (Ql), lower quartile (Q3) and 95% confidence interval (95%CT) were described. T test was adopted to conduct the between-group comparisons. When considering the influence of covariate, the covariance analysis was applied. For time series data, the median survival time was described and its Kaplan-Meier curve was drawn, and the between-group differences in median survival time of the two groups were compared by Log-rank test. When considering center or other influencing factors, the Cox regression analysis was applied.

For qualitative data, the number of cases of various categories and their percentages were described. For counting data comparisons between treatment groups, the chi-square test or Fisher's exact probability test was applied. For grade data comparisons between treatment groups, the Wilcoxon rank-sum test was applied. When considering center or other influencing factors, the CMH chi-square test or logistic regression analysis was applied.

### 8. Intervention effect

### 8.1 The improvement of main symptoms

The results are shown in Tables 1, 2 and 3.

**Table 1. Integral measured value of main symptoms**

| | Visit 1 | Visit 2 | Visit 3 | Statistic | P Value | Test Method |
|---|---|---|---|---|---|---|
| Shortness of breath | | | | | | Mixed-effects model |
| N (Nmiss) | 305(0) | 305(0) | 305(0) | 285.79 | <0.0001 | |
| Mean ± SD | 2.56 ± 1.78 | 1.12 ± 1.37 | 0.16 ± 0.55 | Visit 1 VS 2 | <0.0001 | |
| Median (Q1,Q3) | 2(2,4) | 0(0,2) | 0(0,0) | Visit 1 VS 3 | <0.0001 | |
| Min, Max | 0,6 | 0,6 | 0,2 | Visit 2 VS 3 | <0.0001 | |
| Tiredness and weakness | | | | | | Mixed-effects model |
| N (Nmiss) | 305(0) | 305(0) | 305(0) | 757.19 | <0.0001 | |
| Mean ± SD | 3.38 ± 1.33 | 1.47 ± 1.27 | 0.22 ± 0.67 | Visit 1 VS 2 | <0.0001 | |
| Median (Q1,Q3) | 4(2,4) | 2(0,2) | 0(0,0) | Visit 1 VS 3 | <0.0001 | |
| Min, Max | 0,6 | 0,4 | 0,4 | Visit 2 VS 3 | <0.0001 | |
| Anorexia | | | | | | Mixed-effects model |
| N (Nmiss) | 305(0) | 305(0) | 305(0) | 861.72 | <0.0001 | |
| Mean ± SD | 3.07 ± 1.27 | 1.13 ± 1.18 | 0.05 ± 0.30 | Visit 1 VS 2 | <0.0001 | |
| Median (Q1,Q3) | 4 (2, 4) | 2 (0, 2) | 0 (0, 0) | Visit 1 VS 3 | <0.0001 | |
| Min, Max | 0, 6 | 0, 4 | 0, 2 | Visit 2 VS 3 | <0.0001 | |
| Loose and soft stools | | | | | | Mixed-effects model |
| N (Nmiss) | 305(0) | 305(0) | 305(0) | 140.28 | <0.0001 | |
| Mean ± SD | 1.67 ± 1.69 | 0.62 ± 1.02 | 0.10 ± 0.45 | Visit 1 VS 2 | <0.0001 | |
| Median (Q1,Q3) | 2 (0, 2) | 0 (0, 2) | 0 (0, 0) | Visit 1 VS 3 | <0.0001 | |
| Min, Max | 0, 6 | 0, 4 | 0, 2 | Visit 2 VS 3 | <0.0001 | |

**Table 2. Integral difference value of main symptoms**

| | Difference value of Visit 2-Visit 1 | Difference value of Visit 3-Visit 2 |
|---|---|---|
| Shortness of breath | | |
| N (Nmiss) | 305(0) | 305(0) |
| Mean ± SD | -1.44 ± 1.32 | -2.4 ± 1.8 |
| Median (Q1,Q3) | -2(-2,0) | -2(-4,-2) |
| Min, Max | -6,4 | -6,2 |
| Statistic | -9291.5 | -13225.5 |
| P value | <0.0001 | <0.0001 |
| Test Method | Paired Rank Test | Paired Rank Test |
| Tiredness and weakness | | |
| N (Nmiss) | 305(0) | 305(0) |
| Mean ± SD | -1.91 ± 1.24 | -3.16 ± 1.45 |
| Median (Q1,Q3) | -2(-2,-2) | -4(-4,-2) |
| Min, Max | -6,2 | -6,2 |
| Statistic | -15114 | -20893 |
| P value | <0.0001 | <0.0001 |
| Test Method | Paired Rank Test | Paired Rank Test |
| Anorexia | | |
| N (Nmiss) | 305(0) | 305(0) |
| Mean ± SD | -1.94 ± 1.22 | -3.02 ± 1.29 |
| Median (Q1,Q3) | -2(-2,-2) | -4(-4,-2) |
| Min, Max | -6,2 | -6,2 |
| Statistic | -15609 | -21323.5 |
| P value | <0.0001 | <0.0001 |
| Test Method | Paired Rank Test | Paired Rank Test |
| Loose and soft stools | | |
| N (Nmiss) | 305(0) | 305(0) |
| Mean ± SD | -1.05 ± 1.54 | -1.57 ± 1.65 |
| Median (Q1,Q3) | 0(-2,0) | -2(-2,0) |
| Min, Max | -6,2 | -6,2 |
| Statistic | -4699.5 | -7299 |
| P value | <0.0001 | <0.0001 |
| Test Method | Paired Rank Test | Paired Rank Test |

**Table 3. The disappearance rate of main symptom**

| | Cases with Symptom Resolution at Visit 3 (N) | Proportion (%) | Baseline Symptom-Present Cases (N) |
|---|---|---|---|
| Shortness of breath | 216 | 90 | 240 |
| Tiredness and weakness | 270 | 89.7 | 301 |
| Anorexia | 285 | 97.94 | 291 |
| Loose and soft stools | 161 | 91.48 | 176 |

8.1.1 Symptom of shortness of breath: with the intervention treatment, during the study period, the mean score of visit 2 is decreased by 1.44±1.32 points compared with the baseline score, and the mean score of visit 3 is decreased by 2.4±1.8 points compared with the baseline score. Through the analysis of mixed-effect model and paired rank test, the differences are statistically significant (p<0.05), indicating that the score of the symptom of shortness of breath is decreased significantly after treatment. At the end of the study, the disappearance rate of the symptom of shortness of breath is 90%.

### 8.1.2 Symptom of tiredness and weakness:

with the intervention treatment, during the study period, the mean score of visit 2 is decreased by 1.91±1.24 points compared with the baseline score, and the mean score of visit 3 is decreased by 3.16±1.45 points compared with the baseline score. Through the analysis of mixed-effect model and paired rank test, the differences are statistically significant (p<0.05), indicating that the score of the symptom of tiredness and weakness is decreased significantly after treatment. At the end of the study, the disappearance rate of the symptom of tiredness and weakness is 89.7%.

### 8.1.3 Symptom of anorexia:

with the intervention treatment, during the study period, the mean score of visit 2 is decreased by 1.94±1.22 points compared with the baseline score, and the mean score of visit 3 is decreased by 3.02±1.29 points compared with the baseline score. Through the analysis of mixed-effect model and paired rank test, the differences were statistically significant (p<0.05), indicating that the score of the symptom of anorexia is decreased significantly after treatment. At the end of the study, the disappearance rate of the symptom of anorexia was 97.94%.

### 8.1.3 Symptom of loose and soft stools:

with the intervention treatment, during the study period, the mean score of visit 2 is decreased by 1.05±1.54 points compared with the baseline score, and the mean score of visit 3 is decreased by 1.57±1.65 points compared with the baseline score. Through the analysis of mixed-effect model and paired rank test, the differences are statistically significant (p<0.05), indicating that the score of the symptom of loose and soft stools is decreased significantly after treatment. At the end of the study, the disappearance rate of the symptom of loose and soft stools is 91.48%.

### 8.1 The efficacy results of secondary symptoms

The results are shown in Tables 4, 5 and 6.

**Table 4. Integral measured value of secondary symptoms**

| | Visit 1 | Visit 2 | Visit 3 | Statistic | P Value | Test Method |
|---|---|---|---|---|---|---|
| Vomiting/nausea | | | | | | Mixed-effects model |
| N (Nmiss) | 305 (0) | 305 (0) | 305 (0) | 283.94 | <0.0001 | |
| Mean ± SD | 1.05 ± 0.75 | 0.29 ± 0.48 | 0.01 ± 0.11 | Visit 1 VS 2 | <0.0001 | |
| Median (Q1,Q3) | 1 (1, 2) | 0 (0, 1) | 0 (0, 0) | Visit 1 VS 3 | <0.0001 | |
| Min, Max | 0, 3 | 0, 2 | 0, 1 | Visit 2 VS 3 | <0.0001 | |
| Abdominal distention and fullness | | | | | | Mixed-effects model |
| N (Nmiss) | 305(0) | 305(0) | 305(0) | 217.34 | <0.0001 | |
| Mean ± SD | 0.92 ± 0.73 | 0.23 ± 0.48 | 0.03 ± 0.18 | Visit 1 VS 2 | <0.0001 | |
| Median (Q1,Q3) | 1 (0, 1) | 0 (0, 0) | 0 (0, 0) | Visit 1 VS 3 | <0.0001 | |
| Min, Max | 0, 3 | 0, 2 | 0, 1 | Visit 2 VS 3 | <0.0001 | |
| Weak bowel movements | | | | | | Mixed-effects model |
| N (Nmiss) | 305 (0) | 305 (0) | 305 (0) | 139.49 | <0.0001 | |
| Mean ± SD | 0.78 ± 0.76 | 0.30 ± 0.51 | 0.08 ± 0.26 | Visit 1 VS 2 | <0.0001 | |
| Median (Q1,Q3) | 1 (0, 1) | 0 (0, 1) | 0 (0, 0) | Visit 1 VS 3 | <0.0001 | |
| Min, Max | 0, 3 | 0, 2 | 0, 1 | Visit 2 VS 3 | <0.0001 | |

**Table 5. Integral difference value of secondary symptoms**

| | Difference value of Visit 2-Visit 1 | Difference value of Visit 3-Visit 2 |
|---|---|---|
| Vomiting/nausea | | |
| N (Nmiss) | 305 (0) | 313 (0) |
| Mean ± SD | -0.76 ± 0.68 | -1.04 ± 0.76 |
| Median (Q1,Q3) | -1 (-1, 0) | -1 (-2, -1) |
| Min, Max | -3, 1 | -3, 0 |
| Statistic | -11046 | -13398 |
| P value | <0.0001 | <0.0001 |
| Test Method | Paired Rank Test | Paired Rank Test |

| Abdominal distention and fullness | | |
|---|---|---|
| N (Nmiss) | 305(0) | 305(0) |
| Mean ± SD | -0.70 ± 0.66 | -0.89 ± 0.76 |
| Median (Q1,Q3) | -1 (-1, 0) | -1 (-1, 0) |
| Min, Max | -2, 1 | -3, 1 |
| Statistic | -9146 | -11196.5 |
| P value | <0.0001 | <0.0001 |
| Test Method | Paired Rank Test | Paired Rank Test |

| Weak bowel movements | | |
|---|---|---|
| N (Nmiss) | 305 (0) | 313 (0) |
| Mean ± SD | -0.47 ± 0.67 | -0.70 ± 0.73 |
| Median (Q1,Q3) | 0 (-1, 0) | -1 (-1,0) |
| Min, Max | -3, 1 | -3, 1 |
| Statistic | -4456.5 | -7203.5 |
| P value | <0.0001 | <0.0001 |
| Test Method | Paired Rank Test | Paired Rank Test |

**Table 6. The disappearance rate of secondary symptom**

| | Cases with Symptom Resolution at Visit 3 (N) | Proportion (%) | Baseline Symptom-Present Cases (N) |
|---|---|---|---|
| Vomiting/nausea | 231 | 98.3 | 235 |
| Abdominal distention and fullness | 207 | 97.18 | 213 |
| Weak bowel movements | 159 | 87.85 | 181 |

### 8.2.1 Symptom of vomiting/nausea:

With the intervention treatment, during the study period, the mean score of visit 2 is decreased by 0.76±0.68 points compared with the baseline score, and the mean score of visit 3 is decreased by 1.04±0.76 points compared with the baseline score. Through the analysis of mixed-effect model and paired rank test, the differences are statistically significant (p<0.05), indicating that the score of the symptom of vomiting/nausea is decreased significantly after treatment. At the end of the study, the disappearance rate of the symptom of vomiting/nausea is 98.3%.

### 8.2.2 Symptom of abdominal distention and fullness:

With the intervention treatment, during the study period, the mean score of visit 2 is decreased by 0.7±0.66 points compared with the baseline score, and the mean score of visit 3 is decreased by 0.89±0.76 points compared with the baseline score. Through the analysis of mixed-effect model and paired rank test, the differences are statistically significant (p<0.05), indicating that the score of the symptom of abdominal distention and fullness is decreased significantly after treatment. At the end of the study, the disappearance rate of the symptom of abdominal distention and fullness is 97.18%.

### 8.2.3 Symptom of weak bowel movements:

With the intervention treatment, during the study period, the mean score of visit 2 is decreased by 0.47±0.67 points compared with the baseline score, and the mean score of visit 3 is decreased by 0.7±0.73 points compared with the baseline score. Through the analysis of mixed-effect model and paired rank test, the differences are statistically significant (p<0.05), indicating that the score of the symptom of weak bowel movements is decreased significantly after treatment. At the end of the study, the disappearance rate of the symptom of weak bowel movements is 87.85%.

### 8.3 SF-12 Scale (quality of life assessment scale score) scoring results

The results are shown in Tables 7 and 8.

**Table 7. Measured value of SF12 scale score**

| | Visit 1 | Visit 2 | Visit 3 | Statistic | P Value | Test Method |
|---|---|---|---|---|---|---|
| SF-12 Total Score | | | | | | |
| N (Nmiss) | 305 (0) | 305 (0) | 305 (0) | 290.25 | <0.0001 | Mixed-effects model |
| Mean ± SD | -13.63 ± 8.08 | -4.48 ± 11.44 | 3.42 ± 9.77 | Visit 1 VS 2 | <0.0001 | |
| Median (Q1, Q3) | -14 (-17, -9) | -5 (-12, 3) | 4 (-1, 10) | Visit 1 VS 3 | <0.0001 | |
| Min, Max | -42, 8 | -35, 18 | -32, 20 | Visit 2 VS 3 | <0.0001 | |

**Table 8. Difference value of SF12 scale score**

| | Difference value of Visit 2-Visit 1 | Difference value of Visit 3-Visit 2 |
|---|---|---|
| N (Nmiss) | 305 (0) | 305 (0) |
| Mean ± SD | 9.16 ± 11.59 | 17.06 ± 12.42 |
| Median (Q1, Q3) | 8 (2, 15) | 17 (10, 26) |
| Min, Max | -19, 43 | -18, 47 |
| P value | <0.0001 | <0.0001 |
| Test Method | Paired Rank Test | Paired Rank Test |

With the intervention treatment, during the study period, the mean score of visit 2 is increased by 9.16±11.59 points compared with the baseline score, and the mean score of visit 3 is decreased by 17.06±12.42 points compared with the baseline score. Through the analysis of mixed-effect model and paired rank test, the differences are statistically significant (p<0.05), indicating that the score of SF12 Scale is increased significantly after treatment.

### 9. Conclusion

The Shenling Baizhu can effectively treat various post-healing sequelae of novel coronavirus pneumonia patients, so as to significantly improve the life quality score of patients.

The above are only the optional examples of the present disclosure and are not intended to limit the present disclosure, and for those skilled in the art, the present disclosure may have various modifications and changes. Any modifications, equivalent substitutions and improvements made within the spirit and principles of the present disclosure shall be included in the scope of protection of the present disclosure.

### INDUSTRIAL PRACTICABILITY

The application of Shenling Baizhu in preparing a medicament for treating post-healing sequelae of novel coronavirus pneumonia patients and improving the quality of life is provided in the present disclosure. The sequela is selected from at least one of shortness of breath, fatigue and weakness, inappetence, and diarrhea. The Shenling Baizhu provided by the present disclosure can effectively treat various post-healing sequelae of novel coronavirus pneumonia patients and significantly improve the life quality score of patients, thus having excellent industrial practicability.

## Claims

1. An application of Shenling Baizhu in preparing a medicament for treating a post-healing sequelae of novel coronavirus pneumonia patients and improving quality of life, wherein the sequela is selected from at least one of a shortness of breath, a fatigue and weakness, an inappetence, and a diarrhea; and a raw material of Shenling Baizhu is composed of following parts by weight of Chinese medicinal herbs:
400 parts of ginseng, 400 parts of poria cocos, 400 parts of rhizoma atractylodis macrocephalae stir-fried with bran, 400 parts of Chinese yam, 300 parts of fried white hyacinth beans, 200 parts of lotus seeds, 200 parts of coix seeds stir-fried with bran, 200 parts of fructus amomi, 200 parts of platycodon grandiflorum, and 400 parts of liquorice.

2. The application according to claim 1, wherein the sequelae further comprise at least one selected from a vomiting/nausea, an abdominal distention and fullness, and weak bowel movements.

3. The application according to claim 1, wherein improving the post-healing life quality of novel coronavirus pneumonia patients means that after the Shenling Baizhu is applied to novel coronavirus pneumonia patients, a score of a SF12 scale of the patients is significantly decreased compared with a score before a treatment, and a P value is at least less than 0.05.

4. The application according to any one of claims 1 to 3, wherein the medicament is a clinically acceptable preparation.

5. The application according to claim 4, wherein the medicament is selected from a decoction, a pill, a powder, a granule, a tablet or a capsule.

6. The application according to claim 5, wherein the medicament is a granule and is prepared by following steps, comprising:
I. taking each Chinese medicinal herb according to the parts by weight;
II. pulverizing the Chinese yam into a fine powder, and sieving with a 100-mesh sieve for further utilization;
III. extracting a volatile oil from the fructus amomi by a steam distillation until exhaustion, and collecting a distilled aqueous solution in another container; soaking the spent fructus amomi residue together with the ginseng and rhizoma atractylodis macrocephalae stir-fried with bran in 4-5 times their weight of 95% ethanol for 24 hours, then heating under refluxing for 3 hours, filtering, recovering ethanol from the filtrate, and concentrating to a clear paste with a relative density of 1.00-1.15 at 65°C, so as to obtain an alcohol extract A; reflux-extracting the residue again with 4-5 times their weight of 50% ethanol for 3 hours, filtering, recovering ethanol from the filtrate, and concentrating to a clear paste with a relative density of 1.12-1.13 at 80°C, so as to obtain an alcohol extract B; and reserving the ethanol-extracted residue for further utilization;
IV. decocting poria cocos, fried white hyacinth beans, lotus seeds, coix seeds stir-fried with bran, platycodon grandiflorum, and liquorice with 8-10 times their weight of water for 2 hours, then filtering; combining the residue with the ethanol-extracted residue reserved in Step III, then decocting again with 8 times their weight of water for 1.5 hours, and filtering; combining the two filtrates and the distilled aqueous solution obtained in Step III, standing, and filtering; concentrating the filtrate to a relative density of 1.20 at 80°C, then adding the alcohol extract A and alcohol extract B, and continuing to concentrate into a clear paste with a relative density of 1.30-1.34 at 80°C;
V. blending the fine powder of Chinese yam obtained in step II into the clear paste obtained in step IV, drying at a low temperature of 60°C to obtain a dry paste, and then pulverizing, so as to obtain a medicinal powder; and
VI. taking 1 part by weight of the medicinal powder obtained in step V and 0.4 part by weight of a lactose powder, mixing them evenly, granulating with 70%-75% ethanol; and spraying the volatile oil of fructus amomi obtained in step III, mixing them evenly, and processing into 1000g, so as to obtain a final product.

7. The application according to claim 6, wherein when the granule is applied to a subject in need, a dosage is 3 times a day, 3g per dose, and the dosage is converted into a crude drug dosage of 27.9g/day.
